# EUROPÄISCHE PATENTSCHRIFT

(11) **EP 3 405 240 B1**
(45) Veröffentlichungstag und Bekanntmachung des Hinweises auf die Patenterteilung: **04.11.2020**
(21) Anmeldenummer: 17780987.8
(22) Anmeldetag: 04.07.2017
(51) Int. Cl.: A61M 5/32

(54) **NADEL ZUM DURCHSTECHEN EINER MEMBRAN**
NEEDLE FOR PIERCING A MEMBRANE
AIGUILLE SERVANT À PERCER UNE MEMBRANE

(30) Priorität: 15.07.2016 DE 102016008519; 06.12.2016 DE 102016014445
(43) Veröffentlichungstag der Anmeldung: 28.11.2018
(73) Patentinhaber: Acti-Med AG, 36399 Freiensteinau (DE)
(72) Erfinder: SCHWALB, Andreas, 63636 Brachttal (DE); ZACH, Philip, 36 Eichenzell (DE)
(74) Vertreter: Hase, Stephan
(86) Internationale Anmeldenummer: PCT/DE2017/000192
(87) Internationale Veröffentlichungsnummer: WO 2018/010709

(56) Entgegenhaltungen:
- EP-A1- 0 739 639
- DE-A1- 10 224 101
- DE-A1-102005 027 147
- DE-C1- 4 226 476
- US-A- 2 697 438

## Beschreibung

Die vorliegende Erfindung betrifft eine Nadel zum Durchstechen einer Membran.

Solche Nadeln finden mannigfaltig Anwendung, zum Beispiel in der Pharmazie, in der Medizin, in der Biologie, kurz, überall, wo ein Liquid aus einem mit einer Membran verschlossenen Behältnis entnommen oder dort hinein gegeben werden soll. Dererlei Nadeln bestehen aus einem Nadelrohr (Kanüle), welches innen hohl ist und durch das ein Liquid entweder zu der Nadelspitze hin oder aber zu einem Körper, an den die Nadel angeschlossen ist, geführt wird, und einer Nadelspitze. Die Geometrie der Nadelspitze hat maßgeblichen Einfluss darauf, welcher Kraftaufwand für das Durchstoßen der Membran aufgewandt werden muss und welche Beschädigungen der Membran eintreten. Beim Durchstoßen der Membran mit der Nadelspitze können Partikel der Membran abscheren (sogenanntes "coring") und das im Behälter befindliche Fluid kontaminieren bzw. in die Kanüle eindringen und dort eine Verschmutzung verursachen. Aus diesem Grund ist es gebräuchlich, die Nadelspitze scharfkantig auszubilden und so die Membran beim Einstechen einzuschneiden.

Die US 2,697,438 beschreibt eine Nadel 1 zum Durchstechen einer Membran, welche ein spitz zulaufendes Ende aufweist, welches mit einem Spitzenpunkt 5 zum Durchstechen der Membran und mit einer Öffnung 7 zum Ein- oder Ausleiten einer Flüssigkeit in die oder aus der Nadel versehen ist, wobei das spitz zulaufende Ende einen hinteren Schliff 8 sowie zwei Facettenschliffe 10 besitzt. Die Länge der Facettenschliffe 10 ist länger als 50% der Gesamtlänge des spitz zulaufenden Endes. Der Spitzenpunkt 5 liegt auf der Mittelachse des Kanülenrohrs; er kann, wenn die Unterseite 6 des Kanülenrohrs nicht zum Spitzenpunkt hin gebogen wird, sondern gerade bleibt, auch auf einer Ebene mit der Unterseite 6 des Kanülenrohrs liegen.

Aus der EP 0 819 442 B1 ist eine Nadel zur subkutanen Anwendung wie auch zum Durchstechen von Membranen bekannt, die eine fünfflächige Spitzengeometrie aufweist. An dem zur Kanüle (11) hin gewandten Ende der Nadelspitze (20) ist ein ebener Schliff (30) vorgesehen, an den sich zwei parallel zueinander angeordnete mittlere Facettenschliffe (32a, 32b) anschließen, die den gleichen Schliffwinkel aufweisen wie der ebene Schliff (30). Die mittleren Facettenschliffe (32a, 32b) sind über Schnittstellen (38a, 38b) mit parallel zueinander angeordneten unteren Facettenschliffen (34a, 34b) verbunden, die sich am Spitzenpunkt (36) der Nadelspitze treffen und die einen anderen Schliffwinkel aufweisen als die mittleren Facettenschliffe (32a, 32b). Mit dieser Geometrie soll der Kraftaufwand beim Einstechen der Nadel verringert und damit das Schmerzempfinden des Patienten verringert bzw. die Beschädigung der Membran reduziert werden. Es hat sich jedoch gezeigt, dass durch die Länge (L) der Nadelspitze (20) und die Position des Spitzenpunktes (36) der Nadelspitze (20) auf einer Ebene mit bzw. nahe der unteren Begrenzung der Kanüle (11) beim Durchstechen der Membran eine nicht wünschenswerte Beschädigung derselben erfolgt, wodurch eine Verschmutzung der Kanüle bzw. des Liquids eintreten kann und die Membran so beschädigt wird, dass ein mehrfaches Durchstechen nicht möglich ist, weil die Gefahr des Austretens des Liquids wie auch von dessen Kontamination besteht.

Die EP 1 590 024 B1 beschreibt eine Nadel zum Durchstechen einer Membran, die auf ein Schneiden des Membranmaterials (z.B. Gummi) verzichtet. Die Nadel (1) weist ein spitzes Ende (3) auf, das eine Spitze (4) mit einem Spitzenpunkt (8) zum Durchstechen der Membran (2) besitzt. Die außen liegenden Ränder (19) am spitzen Ende (3) sind vom Spitzenpunkt (8) bis hin zum kanülenseitigen. Rand (25) des spitzen Endes (3) so abgerundet, dass nach dem Durchstechen mit dem Spitzenpunkt (8) das spitze Ende (3) das Material der Membran (2) zur Seite schiebt, anstatt es zu durchschneiden. Dabei kann der Spitzenpunkt (8) auf der Mittelachse der Kanüle liegen, um eine gleichmäßige Dehnung des Membranmaterials zu erreichen. Bei dieser Vorrichtung tritt allerdings das unerwünschte Phänomen auf, dass durch die Dehnung ein Einreißen des Membranmaterials entsteht und so die Membran unkontrollierter beschädigt wird, als dies beim Einschneiden der Fall wäre. Auch hat das spitze Ende (3) der Nadel (1) im Verhältnis zum Kanülendurchmesser eine Länge, die ein relativ langes Dehnen des Membranmaterials erforderlich macht. Das erhöht die Gefahr der Membranbeschädigung und des Austritts von Teilen des Liquids.

Der Erfindung liegt demgemäß die Aufgabe zugrunde, eine Nadel zum Durchstechen einer Membran bereitzustellen, welche ein Einreißen des Membranmaterials verhindert, eine Kontamination des Liquids beim Durchstechen der Membran vermeidet und die Nutzbarkeit der Membran bei wiederholtem Durchstechen erhöht. Diese Aufgabe wird mit den kennzeichnenden Merkmalen des Anspruchs 1 gelöst.

Dadurch, dass der Außendurchmesser des Nadelrohrs 60 bis 70 Prozent der Länge des spitz zulaufenden Endes entspricht, erhält das spitz zulaufende Ende eine kompakte, gedrungene Form. Da die Länge des Facettenschliffs länger ist als 50 Prozent der Gesamtlänge des spitz zulaufenden Endes, hat der Facettenschliff im Vergleich zu bekannten Facettenschliffen eine stark gebogene Form (Löffelform - spoon point Kanüle). Damit kann das spitz zulaufende Ende der Nadel auf vergleichsweise kurzem Weg einen Einschnitt in das Membranmaterial durchführen, der dem tiefsten Punkt der Außenkanten der Facetten und deren höchsten Punkt entspricht. Dabei sind die Außenkanten der Facetten scharfkantig, das heißt, sie sind so scharf, dass sie in das Membranmaterial einschneiden, ohne dass Partikel des Membranmaterials abgeschert werden. Dadurch entsteht ein Schnitt, der das Membranmaterial entspannt und damit den nachfolgenden Dehnvorgang über den hinteren Schliff zur Kanüle hin ohne Einreißen des Membranmaterials ermöglicht. Dieser Dehnvorgang wird auch dadurch erleichtert, dass die Innenkanten der Facettenschliffe gratfrei sind und dass der Spitzenpunkt in einem Bereich angeordnet ist, der auf der Vertikalen von der Längsachse des Nadelrohrs abwärts verläuft und eine Länge hat, die 30 Prozent des Innendurchmessers des Nadelrohrs entspricht. In Verbindung mit der Schnittgeometrie der Facetten wird dadurch der Dehnvorgang des Membranmaterials gleichmäßig.

Nach einer bevorzugten Ausführungsform sind die Innenkanten der Facettenschliffe höher als die Außenkanten, wodurch der Dehnvorgang weiter vereinfacht wird.

Die erfindungsgemäße Vorrichtung soll nachfolgend anhand der Figuren 1 bis 3 näher erläutert werden.

Es zeigen:
- Fig. 1: die erfindungsgemäße Nadel in Seitenansicht
- Fig. 2: die erfindungsgemäße Nadel in Draufsicht
- Fig. 3: das spitz zulaufende Ende der erfindungsgemäßen Nadel in Frontansicht.

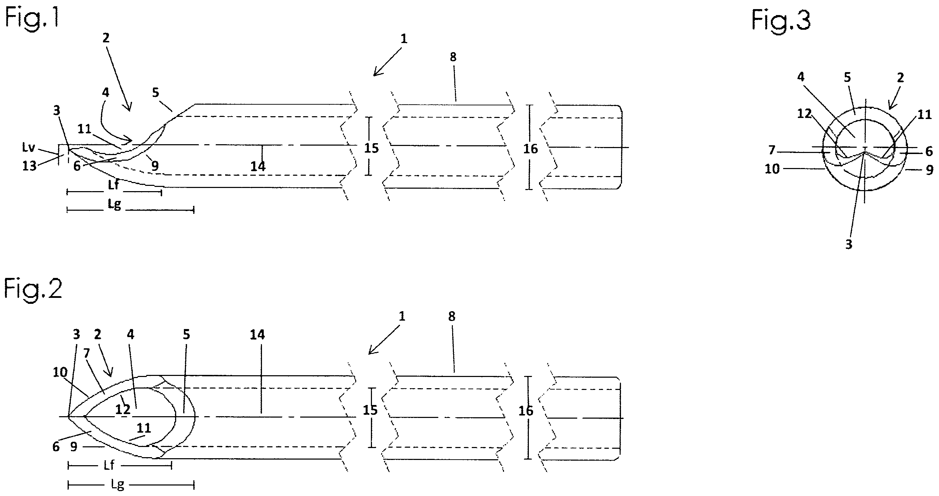

Fig. 1 und 2 zeigen eine Nadel (1) zum Durchstechen einer Membran. Die Nadel (1) besteht aus einem Nadelrohr (8) und einem spitz zulaufenden Ende (2). Das spitz zulaufende Ende (2) setzt sich aus einem hinteren Schliff (5), einer Öffnung (4) zum Ein- oder Ausleiten von Flüssigkeit in das oder aus dem Nadelrohr (8), zwei parallel zueinander angeordneten Facettenschliffen (6, 7) und einem Spitzenpunkt (3) zusammen. Die Außenkanten (9, 10) treffen sich am Spitzenpunkt (3), der in einem Bereich angeordnet ist, der auf der Vertikalen 13 liegt, die von der Mittelachse (14) des Nadelrohrs (8) abwärts verläuft. Die Länge (Lv) der Vertikalen wird dadurch definiert, dass sie 30 Prozent des Innendurchmessers 15 des Nadelrohrs (8) entspricht. Die Länge des spitz zulaufenden Endes (2) wird dadurch bestimmt, dass es 60 bis 70 Prozent des Außendurchmessers 16 des Nadelrohrs (8) entspricht. Durch das Zusammenwirken dieser Maßnahmen wird eine starke Krümmung des spitz zulaufenden Endes (2) erreicht, welches die Form eines Löffels annimmt. Die effiziente Wirkungsweise der Nadel wird dadurch gefördert, dass die Länge (Lf) der Facettenschliffe (6,7) mehr als 50 Prozent der Gesamtlänge (Lg) des spitz zulaufenden Endes (2) beträgt.

Fig. 3 zeigt das spitz zulaufende Ende (2) in Frontansicht. Zu sehen sind die drei Schliffflächen, nämlich der hintere Schliff 5, der rechtsseitige Facettenschliff 7 mit der Außenkante 10 und der höher angeordneten Innenkante 12 sowie der linksseitige Facettenschliff 6 mit der Außenkante 9 und der höher angeordneten Innenkante 11. Die beiden Facettenschliffe treffen sich im Spitzenpunkt (3).

Diese Schliffe umschließen die Öffnung 4.

## Patentansprüche

1. - Nadel (1) zum Durchstechen einer Membran, welche ein spitz zulaufendes Ende (2) aufweist, welches mit einem Spitzenpunkt (3) zum Durchstechen der Membran und mit einer Öffnung (4) zum Ein- oder Ausleiten einer Flüssigkeit in die oder aus der Nadel (1) versehen ist, wobei das spitz zulaufende Ende (2) einen hinteren Schliff (5) sowie zwei Facettenschliffe (6,7) besitzt, wobei die Länge (Lf) der Facettenschliffe (6,7) länger ist als 50 Prozent der Gesamtlänge (Lg) des spitz zulaufenden Endes (2), und wobei der Spitzenpunkt (3) in einem Bereich angeordnet ist, der auf der Vertikalen (13) liegt, die von der Längsachse (14) des Nadelrohrs (8) abwärts verläuft und eine Länge (Lv) hat, die 30 Prozent des Innendurchmessers (15) des Nadelrohrs (8) entspricht,
**dadurch gekennzeichnet,**
**dass**
- der Außendurchmesser (16) des Nadelrohrs (8) 60 bis 70 Prozent der Länge (Lg) des spitz zulaufenden Endes (2) entspricht, und dass
- die Außenkanten (9,10) der Facettenschliffe (6,7) scharfkantig sind, während die Innenkanten (11, 12) gratfrei sind.

2. Nadel (1) zum Durchstechen einer Membran nach Anspruch 1, **dadurch gekennzeichnet,**
**dass** die Innenkanten (11, 12) der Facettenschliffe (6, 7) höher sind als die Außenkanten (9, 10).

## Claims

1. A needle (1) for piercing a membrane, said needle having a pointed end (2) provided with a point (3) for piercing the membrane and with an opening (4) for introducing or discharging a liquid into or out of the needle (1), wherein the pointed end (2) has a rear bevel (5) and two facet bevels (6, 7), wherein the length (Lf) of the facet bevels (6, 7) is longer than 50 percent of the total length (Lg) of the pointed end (2), and wherein the point (3) of the pointed end is located in a region lying on the vertical running (13) down from the longitudinal axis (14) of the needle tube (8) and has a length (Lv)corresponding to 30 percent of the inner diameter (15) of the needle tube (8),
**characterized in that**,
- the outer diameter (16) of the needle tube (8) is 60 to 70 percent of the length (Lg) of the pointed end (2), and that
- the outer edges (9, 10) of the facet bevels (6, 7) are sharp-edged, while the inner edges (11, 12) are burr-free.

2. A needle (1) for piercing a membrane according to claim 1, **characterized in that** the inner edges (11, 12) of the facet bevels (6, 7) are higher than the outer edges (9, 10).

## Revendications

1. Aiguille (1) pour percer une membrane, ladite aiguille ayant une extrémité pointue (2) munie d'une pointe (3) pour percer la membrane et d'une ouverture (4) pour introduire ou déverser un liquide dans ou hors de l'aiguille (1), dont l'extrémité pointue (2) a une coupe rectifée arrière (5) et deux coupes rectifiées à facettes (6, 7), la longueur (Lf) des coupes à facettes (6, 7) étant supérieure à 50 pour cent de la longueur totale (Lg) de l'extrémité pointue (2), et la pointe (3) se trouvant dans une région située sur la verticale (13) descendant de l'axe longitudinal (14) du tube d'aiguille (8) et ayant une longueur (Lv)correspondant à 30 pour cent du diamètre intérieur (15) du tube d'aiguille (8),
**Caractérisée par le fait,**
**que**
- Le diamètre extérieur (16) du tube de l'aiguille (8) est de 60 à 70% de la longueur (Lg) de l'extrémité pointue (2), et que
- les bords extérieurs (9, 10) des coupes à facettes (6, 7) sont à arêtes vives, tandis que les bords intérieurs (11, 12) sont sans bavures.

2. Aiguille (1) pour percer une membrane selon la revendication 1, **caractérisée par le fait que** les bords intérieurs (11, 12) des coupes à facettes (6, 7) sont plus hauts que les bords extérieurs (9, 10).
